# NEW EUROPEAN PATENT SPECIFICATION

(11) **EP 2 592 950 B2**
(45) Date of publication and mention of the opposition decision: **30.11.2022**
(45) Mention of the grant of the patent: 25.04.2018
(21) Application number: 11730593.8
(22) Date of filing: 09.07.2011
(51) Int. Cl.: A23L 33/125, A23L 29/30, A23L 27/30, A23L 2/52, A23L 2/60, A61K 31/7016, A61P 25/00

(54) **ISOMALTULOSE FOR USE IN ENHANCING MENTAL PERFORMANCE**
ISOMALTULOSE ZUR VERWENDUNG BEI DER VERBESSERUNG MENTALER LEISTUNG
ISOMALTULOSE POUR UNE UTILISATION DANS L'AMÉLIORATION DE LA PERFORMANCE MENTALE

(30) Priority: 14.07.2010 EP 10007288
(43) Date of publication of application: 22.05.2013
(73) Proprietor: Südzucker Aktiengesellschaft Mannheim/Ochsenfurt, 68165 Mannheim (DE)
(72) Inventor: HOLUB, Ines, 93049 Regensburg (DE); KOZIANOWSKI, Gunhild, 67269 Grünstadt (DE)
(74) Representative: Schrell, Andreas
(86) International application number: PCT/EP2011/003432
(87) International publication number: WO 2012/007138

(56) References cited:
- EP-A1- 1 393 637
- EP-A1- 1 568 285
- WO-A2-2006/108008
- JP-A- 2000 245 390
- KR-A- 20060 039 766
- KASHIMURA JUN ET AL: "The effect of palatinose on mental concentration in humans.", June 2003 (2003-06), JOURNAL OF NUTRITIONAL SCIENCE AND VITAMINOLOGY, VOL. 49, NR. 3, PAGE(S) 214-216, XP008129518, ISSN: 0301-4800 page 214, paragraph 1 - page 215, right-hand column, paragraph 2; figures 1,2
- ANONYMOUS: "Energy on demand.", INNOVATIONS IN FOOD TECHNOLOGY 2008, no. No. 38, 1 February 2008 (2008-02-01), page 20, XP008129526,
- MARTIN PEARL Y ET AL: "The influence of a glucose drink on a demanding working memory task", 1 August 1999 (1999-08-01), PHYSIOLOGY AND BEHAVIOR, VOL. 67, NR. 1, PAGE(S) 69-74, XP002610921, ISSN: 0031-9384 page 69, paragraph 1-4; figures 1-4

## Description

The present invention relates to isomaltulose for use in enhancing memory in a post-glycemic response phase of a subject in need thereof and a food, drink or pharmaceutical comprising isomaltulose for use in enhancing memory in a post-glycemic response phase.

As glucose is the primary source of energy for the brain, the hypothesis that changes in blood glucose could influence cognitive performance has received a great deal of research interest in recent years. Release of glucose into the blood elicits an insulin response, which serves to return circulating glucose to normal levels and restore homeostasis. The glycemic response or index is a measure of the effects of carbohydrates on blood glucose levels. The glycemic index of a certain food is defined as the area under the two hour blood glucose response curve (AUC) following the consumption of a fixed portion of carbohydrate (usually 50 g). The AUC of the test food is divided by the AUC of the standard (mostly glucose but also white bread, giving two different definitions) and multiplied by 100.

Several studies have investigated the acute effect of manipulation of the peripheral glycemic response on cognitive performance in humans using oral glucose drink interventions accompanied by capillary blood glucose measurements. As a result, improvements in memory or attention have been associated with increased blood glucose levels (glycemic response) in humans. It has therefore been concluded that any relationship between glucose and cognition may be a function of glycemic response and its metabolic consequences. Also, there have been various reports that memory is influenced adversely by missing breakfast or consuming just a small meal. These effects can be reversed by a glucose-containing drink or snack. However, since glucose itself is a rapidly available carbohydrate with a glycemic index of 100, it produces a rapid, spiked and relatively short-term change in blood glucose, which is of little practical importance concerning sustained influence on cognitive or mental performance. Consequently, rapid glycemic load leads to short-term improvements, only (Benton et al., Physiol Behav, 2007, 92, 717-24). As an alternative to modify the glycemic impact of foods, these rapidly digested and thus highly glycemic carbohydrates can be substituted by low glycemic carbohydrates, such as isomaltulose.

Isomaltulose, also termed Palatinose^{™}, is a disaccharide made from sucrose by the enzymatic rearrangement of the alpha-1,2-linkage between glucose and fructose to an alpha-1,6-linkage. It is fully digested and adsorbed in the small intestine and provides the same energy as other sugars. As the adsorption of energy following isomaltulose consumption is much slower than with sucrose and glucose, there is a smaller increase in blood glucose and insulin, allowing energy to be delivered over a prolonged period. Thus, the glycemic index (GI) of isomaltulose is much lower (32) compared to that of sucrose (65) or glucose (100).

Whether isomaltulose has an effect on cognitive performance in humans is still controversial. Dye et al. for example postulated that isomaltulose in a milk-based drink does not affect cognitive performance in healthy adults (Dye et al., Mol Nutr Food Res, 2010, 54, 506-15). Others, see for example EP 1 393 637, have reported that isomaltulose can sustain concentration and attention over a longer time than sucrose does. Two mathematical/arithmetic tests of cognitive performance, namely the Kraepelin test and the sequential memory test were performed by subjects at 90 minutes and at 150 minutes following the ingestion of isomaltulose. At 150 minutes pro-bands that ingested the isomaltulose-containing drink performed significantly better in the cognitive performance tests than the ones that ingested sucrose-containing drinks. This effect can be explained by the prolonged and slower increase in blood glucose levels, providing just sufficiently elevated glucose levels at 150 minutes after consumption, such as evident from Holub et al. (Br J Nutr, 2010, 1730-37).

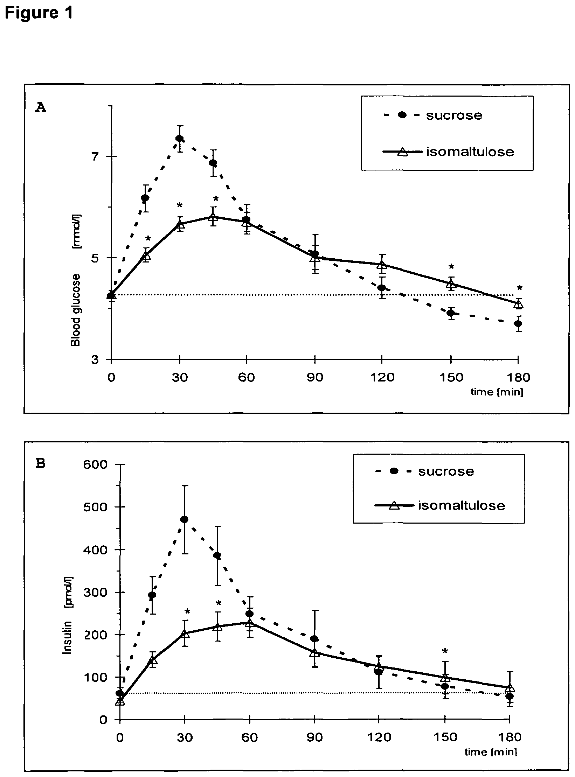

Said document examines blood glucose and insulin levels after the ingestion of 50 g isomaltulose and sucrose (see figure 1). The results show that after ingestion of isomaltulose the blood glucose or glycemic response curve is much lower than the one after ingestion of sucrose. In contrast to the sucrose evoked glycemic response blood glucose levels caused by isomaltulose increase gradually and only moderately whereby a plateau rather than a peak is obtained. After the maximum, blood glucose slowly declines and approximates the initial levels about 2.5 hours after the ingestion, which marks the end of the glycemic response phase and the beginning of a post-glycemic response phase. With the ending of the glycemic response phase, however, no effect on mental performance caused by the uptake of isomaltulose can be expected anymore. KASHIMURA JUN ET AL: "The effect of palatinose on mental concentration in humans.", June 2003 (2003-06), Journal of Nutritional Science and, VOL. 49, NR. 3, PAGE(S) 214-216 discloses the use of isomaltulose for enhancing mental performance over a longer time than sucrose does. There is no effect on enhanced mental performance suggested or discussed, when blood glucose levels are no longer elevated. Therefore, the technical problem underlying the present invention is to provide means to enhance memory not dependent on an elevated blood glucose level or glycemic response caused for example by ingestion of a meal or snack, in particular means to sustain or enhance memory in a post-glycemic response phase.

The present invention solves its underlying technical problem according to the teaching of the independent claims. Accordingly, the present invention provides isomaltulose for use in enhancing memory in a post-glycemic response phase of a subject in need thereof. Thus, the present invention provides a method for enhancing or sustaining memory in a post-glycemic response phase of a subject in need thereof by administering isomaltulose to a subject in need thereof, in particular in an amount effective to achieve said effect. The present invention provides the use of isomaltulose for enhancing memory in a post-glycemic response phase of a subject in need thereof.

Surprisingly, it could be shown that isomaltulose compared to high Glycemic Index (GI) food enhances memory and attention, in a post-glycemic response phase, i. e. at a time point when blood glucose levels already had approximated initial values, for example at 〉 150 minutes, in particular 160 minutes, preferably 180 minutes past the consumption of isomaltulose.

Thus, the present invention provides the unexpected and advantageous teaching that isomaltulose is able to enhance memory in a very late phase after consumption of isomaltulose or, for instance, an isomaltulose containing food or drink when compared to a high GI counterpart. The present teaching provides the use of isomaltulose in enhancing memory in a phase occurring subsequent to a glycemic response phase of a subject wherein said glycemic response phase is caused by consumption of said isomaltulose. Thus, the invention is inter alia advantageous in so far, as it teaches the beneficial use of isomaltulose for enhancing memory in a time phase far distant from the consumption of isomaltulose and therefore provides a prolonged higher memory of the subject. Hence, the present invention enables the subject to memorize on a high level for a longer time period, in particular in an extra late phase, than possible with high GI food, i.e. a source of rapidly available glucose, e.g. maltodextrins, sucrose, glucose or processed starches. It could be shown by the inventors that memory and attention is enhanced, intensified or beneficially affected in an extra late phase by the uptake of isomaltulose, i.e. in a phase where blood glucose levels are no longer elevated subsequent to the ingestion of isomaltulose, for instance in form of a food containing isomaltulose. The same extended effect is not seen with high GI foods even though comparable effects in the glycemic response phase were observed. In a particularly preferred embodiment of the present invention isomaltulose is used for enhancing memory in a post-glycemic response phase, wherein the post-glycemic response phase is an extra late phase after the consumption of isomaltulose, for instance in form of a food containing isomaltulose.

According to the present invention, initial values of the blood glucose level are those which are present before the consumption, of isomaltulose, in particular are values which reflect the blood glucose level in a subject not changed by or directly reflecting the consumption of a meal, that means are values where the blood glucose level does not show any meal-responsive glycemic characteristic.

In the context of the present invention the term "post-glycemic response phase" means a phase occurring after the glycemic response phase which itself is characterised by the presence of elevated blood glucose levels, being caused by and occurring subsequent to a carbohydrate-containing meal, hereinafter also called snack. In the post-glycemic response phase the blood glucose level has approximated its initial value, which caused the glycemic response. Thus, in the context of the present invention the post-glycemic response phase is a phase subsequent to a glycemic response phase which is caused by and therefore subsequent to the consumption of the isomaltulose or isomaltulose-containing food, drink or pharmaceutical. Hence, in the context of the present invention the post-glycemic response phase is characterised by blood glucose levels that have approximated to +/- 15 % of the initial values of blood glucose in the preglycemic phase, i. e. before the isomaltulose consumption, hereinafter also called uptake or ingestion. In the context of the present invention the term "post-glycemic response phase" in particular means a "late postprandial response phase" also called a "late postprandial phase". In the context of the present invention the term "glycemic response phase" in particular means an "early postprandial response phase" also called an "early postprandial phase".

In the context of the present invention the term "enhancing memory" means an improvement in memory due to the consumption of isomaltulose compared to a high GI food at the same time, wherein the enhancement is present in the post-glycemic response phase of a subject in need thereof and wherein the enhancement is an enhancement in comparison to the level of memory present in the subject after the consumption of high GI food.

An enhancement of memory in accordance with the present invention is determined by measuring the memory level of a statistically relevant number of subjects by using test procedures well-known in the art for determining memory and attention subsequent to consumption of isomaltulose and for comparison subsequent to consumption of high GI food, in particular in the post-glycemic response phase.

The method, recall of objects, is well-known to assess episodic memory and the spatial recall of objects is established to determine spatial memory (Elliott CD, British Ability Scales, 1996, National Foundation of Education Research; Isaacs & Oates, Eur J Nutr, 2008, 47 (Suppl 3), 4-24). Signal detection tests, e.g. the Paradigm of Shakow are well-known to measure the attention performance (Kallus et al., Eur J Nutr, 2005, 44, 465-84; Shakow, Arch general Psychiat, 1962, 6, 1-17). The assessment of reaction time by measuring the response speed to light stimuli is often used and established (Schmitt et al., Eur J Nutr, 2005, 44, 459-64). Assessing the speed for monotonous item selection is known as a method to determine speed of information processing (Elliott CD, British Ability Scales, 1996, National Foundation of Education Research).

In a particularly preferred embodiment of the present invention an enhancement of memory can be a sustainment of memory, that means in this preferred embodiment, the isomaltulose is used to sustain memory in a post-glycemic response phase of a subject in need thereof, wherein the sustainment is defined by comparing the level of memory in the post-glycemic response phase of a subject in need thereof after consumption of isomaltulose with the memory level of said subject in the glycemic response phase after consumption of isomaltulose, while in those subjects eating high GI food memory declined from the glycemic to post-glycemic response phase.

The term "high GI food" in accordance with the present invention means a product or substance mixture in solid, liquid, dissolved or suspended form with a Glycemic Index (GI) higher than 55, while the term "low GI" characterises a Glycemic Index (GI) equal or less than 55.

In a preferred embodiment of the present invention, memory is considered to be enhanced by the consumption of isomaltulose in a post-glycemic response phase occurring directly after a glycemic response phase subsequent to consumption of said isomaltulose, wherein in said post-glycemic response phase blood glucose levels have returned to +/- 15 %, preferably +/- 10 %, of the initial value of before the consumption of said isomaltulose.

In a particularly preferred embodiment of the present invention memory enhanced by isomaltulose is selected from the group consisting of episodic memory and spatial memory most preferably episodic memory.

In a preferred embodiment of the present invention the post-glycemic response or extra late phase is from 〉 150 minutes to six hours, preferably from 151 minutes to six hours, preferably from 152 minutes to six hours, preferably from 155 minutes to six hours, particularly preferred from 160 minutes, more particularly 180 minutes to six hours after consumption of the isomaltulose by the subject in need thereof.

In the context of the present invention the term "subject in need thereof' refers to animals or humans. In a preferred embodiment of the present invention a subject in need thereof is a healthy, in particular a mentally healthy human. The present use of isomaltulose is in a preferred embodiment a non-medical use. However, in another preferred embodiment, the use of isomaltulose is a medical use, in particular relating to mentally ill or disturbed subjects, in particular humans. Accordingly, the present invention also relates to the use of isomaltulose to prepare a pharmaceutical composition for enhancing memory in a post-glycemic response phase of a subject in need thereof.

In a preferred embodiment of the present invention isomaltulose is to be consumed by the subject in need thereof in a dose from 0.1 g/kg to 1.7 g/kg body weight, preferably from 0.2 g/kg to 1.5 g/kg body weight, most preferably from 0.3 g/kg to 1.0 g/kg body weight.

In a preferred embodiment of the present invention isomaltulose can also be used together with other sweetening agents selected from the group of sugars and sugar alcohols, preferably trehalulose, isomalt, maltitol, xylitol, erythritol, sucromalt, trehalose, tagatose or mannitol.

In a preferred embodiment of the present invention the isomaltulose may also be combined with high intense sweeteners, such as acesulfam K, aspartame, stevia derived extracts, thaumatin, monellin, sucralose, dihydrochalkon, cyclamat or saccharin.

Preferred according to the invention is the use of isomaltulose as specified in the invention either alone or in a mixture of sweeteners, preferably a mixture of sweeteners containing isomaltulose and at least one high intense sweetener, especially sucralose. Especially preferably, the mixture of sweeteners is comprised of only isomaltulose and sucralose; optionally, however, additional components may also be included, for example additional sweetening agents.

Preferred according to the invention is the use of isomaltulose alone as specified in the invention or in a mixture of sweeteners, wherein 1 to 99 % by weight, especially 40 to 99 % by weight, preferably 70 to 98 % by weight, more preferably 80 to 95 % by weight of isomaltulose is used together with other sweeteners in a mixture (based on total dry substance weight of the mixture of sweeteners).

Preferred according to the invention is the use of isomaltulose as specified in the invention, especially in the aforementioned quantities, in a mixture of sweeteners, wherein the mixture of sweeteners contains 0.0005 to 3 % by weight, especially 0.001 to 1 % by weight, preferably 0.01 to 0.5 % by weight, more preferably 0.02 to 0.1 % by weight high intense sweetener (based on the total dry substance weight of the mixture of sweeteners).

In a particularly preferred embodiment of the present invention isomaltulose is used in a food, in particular semi-luxury food or confectionary, beverage, in particular a drink, or pharmaceutical to enhance memory in a post-glycemic response phase of a subject in need thereof. The present invention also relates to a food, in particular semi-luxury food or confectionary, beverage, in particular a drink or pharmaceutical for use in enhancing memory in a post-glycemic response phase of a subject in need thereof comprising isomaltulose.

In a preferred embodiment of the present invention the isomaltulose, in particular the isomaltulose-containing food, beverage or pharmaceutical, is used according to the present invention, wherein the isomaltulose is the only sweetening agent used, preferably wherein the isomaltulose is the only sugar used, in particular wherein the isomaltulose is the only body-providing sweetening agent used according to the present invention. In a particularly preferred embodiment of the present invention the isomaltulose, in particular the isomaltulose-containing food, drink or pharmaceutical, is used according to the present invention, wherein the isomaltulose is used without glucose and/or without fructose and/or without sucrose and/or without lactose and/or without intense sweeteners. In the context of the present invention, the term "sweetening agent" is used to refer to substances that have sweetening power and are added, for example, to foods or beverages in order to give them a sweet taste. In the context of the present invention "sweetening agents" are subdivided into "sugars" such as isomaltulose, saccharose, glucose or fructose, which provide body as well as at least one of calories and energy and sweetening power, and "sweeteners", in other words substances that are not sugars but nevertheless have sweetening power, which are in turn subdivided into "sugar substitutes", in other words sweetening agents that have body and a physiological calorific value in addition to a sweetening power (sweeteners absorbed by the body), and "intense sweeteners", in other words substances that ordinarily have a very high sweetening power without providing at least one of calories and energy.

Preferred according to the invention is the use of isomaltulose as specified in the invention in a dietetic food. Also preferred according to the invention is the use of a mixture of sweeteners containing isomaltulose in a dietetic food.

Preferred according to the invention is the use of isomaltulose alone or in a mixture of sweeteners in a beverage, food or pharmaceutical, wherein the beverage, food or pharmaceutical contains 1 to 99 % by weight, especially 20 to 70 % by weight, preferably 30 to 60 % by weight, more preferably 40 to 55 % by weight isomaltulose alone or in a mixture with other sweeteners (based on the total dry substance weight of the beverage, food or pharmaceutical). In a particular embodiment, the beverage, food or pharmaceutical does not contain glucose, fructose and/or saccharose. According to the invention, however, an embodiment may contain glucose, fructose, saccharose and/or other sweetening agents.

The aforementioned beverages, include for example, non-alcoholic beverages, refreshment beverages, cola-containing beverages, sports drinks, beverages ingredients, and drink powders.

In the context of the present invention, the term "foods" refers to products or substance mixtures in solid, liquid, dissolved or suspended form that are used predominantly to nourish humans and are consumed by humans in an unaltered, prepared or processed form. Foods may contain other components in addition to their natural constituents, which may be of natural or synthetic origin. Foods may be in solid form or in liquid form. The term "semi-luxury foods" or "confectionary" refers predominantly to substances or mixtures of substances, in solid, liquid, dissolved or suspended form, that provide enjoyment to the human or animal body when consumed.

In the preferred embodiment of the invention, the foods mentioned in the invention refer to milk or milk products, such as cheese, butter, yogurt, kefir, quark, sour milk, buttermilk, cream, condensed milk, freeze-dried milk, whey, milk mixtures, milk half-fat, whey mixture products, milk sugar, milk protein and milk fat products. In a further preferred embodiment of the invention, the foods mentioned in the invention refer to baked goods, especially breads including cookies and cakes, and including preserved baked goods. In further embodiments of the invention, the foods mentioned in the invention refer to bread spreads, margarine products and shortening, as well as instant products and broths. In further embodiments of the invention, the foods mentioned in the invention refer to fruit products, especially jams, marmalades, jellies, canned fruits, fruit pulp, fruit juices, fruit juice concentrates, fruit nectar, and powdered fruit. The foods containing the products according to the invention can also be vegetable products, especially canned vegetables, vegetable juices and vegetable pulp, according to the invention.

The term semi-luxury foods refers, for example, to confections, especially chocolate products, hard caramels, soft caramels, fondant products, gelled products, licorice, whipped sugar products, flaked coconut, dragees, condensed foods, candied fruits, brittle, nougat products, frozen confections, marzipan, chewing gum, granola bars, and ice creams, or alcoholic or non-alcoholic sweetened drinks.

In the context of the present invention, "dietetic foods" are understood as foods that are intended to serve a specific nutritional purpose, in that they affect the supply of certain nutrients or other nutritional substances that produce a physiological effect in a certain proportion or in a certain condition. Dietetic foods differ substantially from foods of a comparable type in their composition or in their properties. Dietetic foods can be used in all cases in which certain nutritional requirements must be fulfilled due to illnesses, functional disorders or allergic reactions to specific foods or their ingredients. Dietetic foods can be in solid or liquid form.

According to the invention, a beverage, food or pharmaceutical as specified in the invention preferably contains 1 to 99 % by weight, especially 20 to 70 % by weight, preferably 30 to 60 % by weight, more preferably 40 to 55 % by weight isomaltulose alone or in a mixture with other sweeteners according to the invention (based on the total dry substance weight of the beverage, food, semi-luxury food or pharmaceutical).

The food comprising isomaltulose might be selected from the group consisting of cereals, milk products, confectionaries, chewing gums, bakery products and jellies.

The pharmaceutical comprising isomaltulose is preferably in form of tablets, capsules, granules, powder, an ingestible solution or an injectable solution.

The beverage, in particular drink, comprising isomaltulose might be selected from the group consisting of a soft drink, water-based drink, sports drink, jelly drink, tea drink, cocoa drink, coffee drink, alcoholic drink, energy drink, fruit drink or fun drink. The term "water-based drink" refers to drinks that are mainly consisting of water, but containing other ingredients as well. The term "fun drink" in the context of the present invention refers to drinks that might be coloured or otherwise modified, for example by addition of specific ingredients, to appeal especially to younger consumers.

Other preferred embodiments are the subject matter of the subclaims.

In the following, the invention is described in detail by way of examples and the accompanying figures.

The figures show:
- Figure 1 shows the blood glucose and insulin response after the uptake of either isomaltulose or sucrose over time. (a) blood glucose profile, (b) insulin profile, data were collected from ten healthy subjects. * mean values were significantly different by Wilcoxon test for paired data. Dotted lines mark initial values of blood glucose and insulin levels.

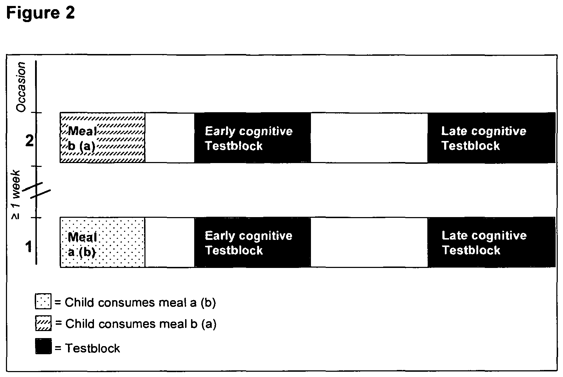

- Figure 2 shows an overview of the test procedure over time.

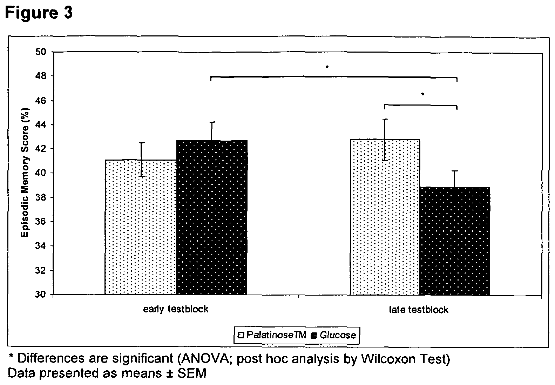

- Figure 3 shows the results of the episodic memory test after consumption of an isomaltulose or glucose sweetened meal at an early and late postprandial cognitive test block. The two meals did not differ in the early postprandial phase (early cognitive test block), but in the late postprandial phase, i.e. 〉 150 minutes (late cognitive test block) those eating the isomaltulose rather than glucose sweetened meals had significantly better episodic memories. Between the early and the late postprandial phase the episodic memory significantly declined in those eating the glucose meal but not in those eating the isomaltulose meal. Data are represented as means ± SEM.

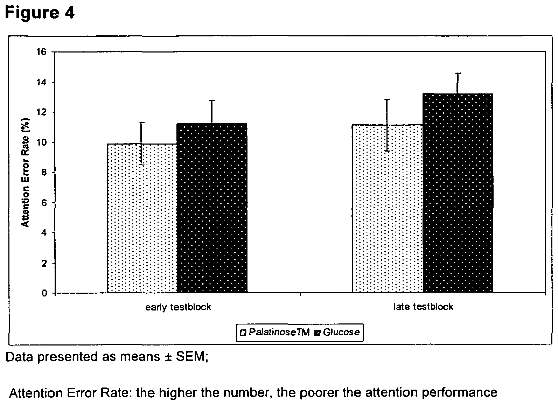

- Figure 4 shows the results of the attention test after consumption of an isomaltulose or glucose sweetened meal in an early and late postprandial phase. The given attention error rate means the higher the number, the poorer the attention performance. In the early postprandial phase (early cognitive test block) both meals induced nearly the same attention error rate. At the late cognitive testing (late postprandial phase, i.e. 〉 150 minutes) children who consumed the isomaltulose sweetened meal performed better by tendency compared to children who consumed the high GL meal with glucose. Comparing the early and the late postprandial phase there was a higher increase in error rate in those eating the glucose meal compared to those eating the isomaltulose meal. Data are represented as means ± SEM.

### Example 1 Isomaltulose sustains memory and attention in a post-glycemic response phase in children Procedure

Seventy-five healthy school children consumed in crossover design two meals designed to differ in their glycemic load (GL; Amount of carbohydrate x Glycemic Index, GI, of food items; GI of isomaltulose = 32, GI of glucose = 100) while being similar in macro-nutrient content. The cognitive test block takes 45 minutes and was once assessed in the early postprandial phase and once in the late postprandial phase (〉 150 minutes) after meal ingestion. Every child participated in four blocks of testing ( Figure 2 ).
- Early test block after meal A
- Late test block after meal A
- Early test block after meal B
- Late test block after meal B

On each cognitive test block parallel versions of the cognitive tests were administered.

### Meals

Both meals offered a similar intake of energy (1385 KJ) and macro-nutrients (84.4 % carbohydrates, 10.6 % protein and 5.0 % fat) while differing in glycemic load (GL; Amount of carbohydrate x Glycemic Index of food items), that is whether glucose was released slowly or quickly into the blood stream. The meals were composed as followed (% of total energy):
- High GL meal: Cornflakes + milk (35.3 %), yoghurt + fruit preparation (14.2 %), glucose (50.5 %).
- Low GL meal: Cornflakes + milk (35.3 %), yoghurt + fruit preparation (14.2 %), isomaltulose (50.5 %).

### Episodic memory test

The approach was based on the Recall of Objects test of the British Ability Scales (Elliott, British Ability Scales, 1996, National foundation of Educational Research) and children had to recall up to 60 objects.

### Results for episodic memory

The results are shown in Figure 3. When episodic memory was assessed in the early postprandial phase after meal ingestion it was similar in the two groups. However, in the late postprandial phase (〉 150 minutes after meal consumption) those who consumed isomaltulose had better episodic memories compared to children who consumed the higher GL glucose meal.

After eating the higher GL meal (containing glucose) episodic memory was significantly lower in the late postprandial phase compared to the early postprandial phase.

After eating the lower GL meal (containing isomaltulose) the episodic memory tested in the late postprandial phase as opposed to early postprandial phase after meal ingestion was not significantly different; in fact there was a trend for it to be slightly better in the post-glycemic or late postprandial response phase.

### Attention test

The paradigm of Shakow was used to assess attention with error rate as outcome (Shakow, Arch general Psychiat, 1962, 6, 1-17). In this signal detection test the children had to respond as quickly as possible to a delayed light stimulus.

### Results of attention test

The results are presented in Figure 4. The both meals induced nearly the same error rates in the early postprandial phase. In the late postprandial phase (〉 150 minutes after meal ingestion) there was a trend for better attention performance (i.e. lower error rate) with the isomaltulose meal compared to the glucose meal. The absolute percentage increase in error rate was higher in the glucose group than in the isomaltulose group and indicated a better attention performance with isomaltulose opposed to the high GL meal containing glucose in the late postprandial phase.

The present study shows that an isomaltulose containing meal positively affects episodic memory, and attention, when compared to a classically sweetened meal. The effect was pronounced in the late postprandial phase, from 〉 150 minutes after meal ingestion onwards, which is a surprising observation since this time point is in the post-glycemic or late postprandial response phase.

## Claims

1. Non-therapeutic use of isomaltulose for enhancing memory in a post-glycemic response phase of a healthy human subject in need thereof, wherein the post-glycemic response phase is from 180 minutes to 6 hours after consumption of the isomaltulose by the subject in need thereof and is **characterized by** blood glucose levels which are no longer elevated subsequent to the ingestion of isomaltulose.

2. The use according to claim 1, wherein the enhancement is an enhancement of memory in comparison to the level of memory in a post-glycemic phase after consumption of high glycemic index food.

3. The use according to any one of the preceding claims, wherein the memory is selected from the group consisting of episodic memory and spatial memory.

4. The use according to any one of the preceding claims, wherein the memory of children, adolescents and adults is enhanced.

5. The use according to any one of the preceding claims, wherein the dose of isomaltulose to be consumed by the subject in need thereof is from 0.1 g/kg to 1.7 g/kg body weight.

6. The use according to any one of the preceding claims, which is used with another sweetening agent selected from the group of sugars, sugar alcohols and high intense sweeteners.

7. The use according to any one of the preceding claims, which is used in a food, beverage or pharmaceutical.

8. Non-therapeutic use of a food or beverage comprising isomaltulose for enhancing memory in a post-glycemic response phase of a healthy human subject in need thereof, wherein the post-glycemic response phase is from 180 minutes to 6 hours after consumption of the isomaltulose by the subject in need thereof and is **characterized by** blood glucose levels which are no longer elevated subsequent to the ingestion of isomaltulose.

9. The use according to claim 8, wherein the beverage is selected from the group consisting of a soft drink, water-based drink, sports drink, jelly drink, tea drink, cocoa drink, coffee drink, alcoholic drink, energy drink, fruit drink or fun drink.

10. The use according to claims 8 or 9, wherein the food is selected from the group consisting of cereals, milk products, confectionaries, chewing gums, bakery products and jellies.

11. Use of isomaltulose for the preparation of a pharmaceutical composition for use in a method for enhancing memory in a post-glycemic response phase of a mentally ill or disturbed subject in need thereof, wherein the post-glycemic response phase is from 180 minutes to 6 hours after consumption of the isomaltulose by the subject in need thereof and is **characterized by** blood glucose levels which are no longer elevated subsequent to the ingestion of isomaltulose.

12. Use of a pharmaceutical comprising isomaltulose for the preparation of a pharmaceutical composition for use in a method for enhancing memory in a post-glycemic response phase of a mentally ill or disturbed subject in need thereof, wherein the post-glycemic response phase is from 180 minutes to 6 hours after consumption of the isomaltulose by the subject in need thereof and is **characterized by** blood glucose levels which are no longer elevated subsequent to the ingestion of isomaltulose.

## Patentansprüche

1. Nicht-therapeutische Verwendung von Isomaltulose zur Verbesserung des Gedächtnisses in einer postglykämischen Reaktionsphase eines gesunden menschlichen Subjekts, das einen entsprechenden Bedarf aufweist, wobei die postglykämische Reaktionsphase von 180 Minuten bis 6 Stunden nach dem Verzehr der Isomaltulose durch das Subjekt, das einen entsprechenden Bedarf aufweist, beträgt und durch Blutglukosespiegel gekennzeichnet ist, die nach der Einnahme von Isomaltulose nicht mehr erhöht sind.

2. Verwendung nach Anspruch 1, wobei die Verbesserung eine Verbesserung des Gedächtnisses im Vergleich zu dem Niveau des Gedächtnisses in einer postglykämischen Phase nach dem Konsum eines Nahrungsmittels mit hohem glykämischem Index ist.

3. Verwendung nach einem der vorhergehenden Ansprüche, wobei das Gedächtnis ausgewählt ist aus der Gruppe bestehend aus episodischem Gedächtnis und räumlichem Gedächtnis.

4. Verwendung nach einem der vorhergehenden Ansprüche, wobei das Gedächtnis von Kindern, Jugendlichen und Erwachsenen verbessert wird.

5. Verwendung nach einem der vorhergehenden Ansprüche, wobei die Dosis Isomaltulose, die von dem Subjekt, das einen entsprechenden Bedarf aufweist, konsumiert werden sollte, von 0,1 g/kg bis 1,7 g/kg Körpergewicht beträgt.

6. Verwendung nach einem der vorhergehenden Ansprüche, zusammen mit einem weiteren Süßstoff ausgewählt aus der Gruppe der Zucker, Zuckeralkohole und Intensivsüßstoffe.

7. Verwendung nach einem der vorhergehenden Ansprüche, in einem Nahrungsmittel, Getränk oder Pharmazeutikum.

8. Nicht-therapeutische Verwendung eines Nahrungsmittels oder Getränks umfassend Isomaltulose zur Verbesserung des Gedächtnisses in einer postglykämischen Reaktionsphase eines gesunden menschlichen Subjekts, das einen entsprechenden Bedarf aufweist, wobei die postglykämische Reaktionsphase von 180 Minuten bis 6 Stunden nach dem Verzehr der Isomaltulose durch das Subjekt, das einen entsprechenden Bedarf aufweist, beträgt und durch Blutglukosespiegel gekennzeichnet ist, die nach der Einnahme von Isomaltulose nicht mehr erhöht sind.

9. Verwendung nach Anspruch 8, wobei das Getränk ausgewählt ist aus der Gruppe bestehend aus Erfrischungsgetränken, wasserbasierten Getränken, Sportgetränken, angedickten Getränken, Teegetränken, Kakaogetränken, Kaffeegetränken, alkoholischen Getränken, Energiegetränken, Fruchtgetränken oder Fun-Getränken.

10. Verwendung nach den Ansprüchen 8 oder 9, wobei das Nahrungsmittel ausgewählt ist aus der Gruppe bestehend aus Cerealien, Milchprodukten, Süßwaren, Kaugummis, Backprodukten und Gelees.

11. Verwendung von Isomaltulose zur Herstellung einer pharmazeutischen Zusammensetzung zur Verwendung in einem Verfahren zur Verbesserung des Gedächtnisses in einer postglykämischen Reaktionsphase eines psychisch kranken oder gestörten Subjekts, das einen entsprechenden Bedarf aufweist, wobei die postglykämische Reaktionsphase von 180 Minuten bis 6 Stunden nach dem Verzehr der Isomaltulose durch das Subjekt, das einen entsprechenden Bedarf aufweist, beträgt und durch Blutglukosespiegel gekennzeichnet ist, die nach der Einnahme von Isomaltulose nicht mehr erhöht sind.

12. Verwendung eines Isomaltulose enthaltenden Pharmazeutikums zur Herstellung einer pharmazeutischen Zusammensetzung zur Verwendung in einem Verfahren zur Verbesserung des Gedächtnisses in einer postglykämischen Reaktionsphase eines psychisch kranken oder gestörten Subjekts, das einen entsprechenden Bedarf aufweist, wobei die postglykämische Reaktionsphase von 180 Minuten bis 6 Stunden nach dem Verzehr der Isomaltulose durch das Subjekt, das einen entsprechenden Bedarf aufweist, beträgt und durch Blutglukosespiegel gekennzeichnet ist, die nach der Einnahme von Isomaltulose nicht mehr erhöht sind.

## Revendications

1. Utilisation non thérapeutique de l'isomaltulose pour améliorer la mémoire dans une phase de réponse post-glycémique chez un sujet humain sain qui en a besoin, dans laquelle la phase de réponse post-glycémique est de 180 minutes à 6 heures après la consommation de l'isomaltulose par le sujet qui en a besoin et est **caractérisée par** des niveaux de glucose dans le sang qui ne sont plus élevés après l'ingestion de l'isomaltulose.

2. Utilisation selon la revendication 1, dans laquelle l'amélioration est une amélioration de la mémoire par rapport au niveau de mémoire dans une phase post-glycémique après la consommation des aliments à indice glycémique élevé.

3. Utilisation selon l'une quelconque des revendications précédentes, dans laquelle la mémoire est choisie dans le groupe consistant en la mémoire épisodique et la mémoire spatiale.

4. Utilisation selon l'une quelconque des revendications précédentes, dans laquelle la mémoire des enfants, des adolescents et des adultes est améliorée.

5. Utilisation selon l'une quelconque des revendications précédentes, dans laquelle la dose de l'isomaltulose à être consommée par le sujet qui en a besoin est dans la rangée de 0,1 g/kg à 1,7 g/kg du poids corporel.

6. Utilisation selon l'une quelconque des revendications précédentes, avec un autre agent édulcorant choisi dans le groupe des sucres, des alcools de sucre et des édulcorants intenses.

7. Utilisation selon l'une quelconque des revendications précédentes, dans un aliment, une boisson ou un produit pharmaceutique.

8. Utilisation non-thérapeutique d'un aliment ou d'une boisson comprenant de l'isomaltulose pour améliorer la mémoire dans une phase de réponse post-glycémique chez un sujet humain sain qui en a besoin, dans laquelle la phase de réponse post-glycémique est de 180 minutes à 6 heures après la consommation de l'isomaltulose par le sujet qui en a besoin et est **caractérisée par** des niveaux de glucose dans le sang qui ne sont plus élevés après l'ingestion de l'isomaltulose.

9. Utilisation selon la revendication 8, la boisson étant choisie dans le groupe consistant en des boissons rafraîchissantes, des boissons à base d'eau, des boissons pour sportifs, des boissons gelées, des boissons à base de thé, des boissons à base de cacao, des boissons à base de café, des boissons alcoolisées, des boissons énergisantes, des boissons à base de fruits ou des boissons fun.

10. Utilisation selon les revendications 8 ou 9, l'aliment étant choisi dans le groupe consistant en céréales, produits laitiers, confiseries, gommes à mâcher, produits de boulangerie et gelées.

11. Utilisation d'isomaltulose pour la préparation d'une composition pharmaceutique destinée à être utilisée dans un procédé pour améliorer la mémoire dans une phase de réponse post-glycémique d'un sujet malade ou perturbé mentalement qui en a besoin, dans laquelle la phase de réponse post-glycémique est de 180 minutes à 6 heures après la consommation de l'isomaltulose par le sujet qui en a besoin et est **caractérisée par** des niveaux de glucose dans le sang qui ne sont plus élevés après l'ingestion de l'isomaltulose.

12. Utilisation d'un produit pharmaceutique comprenant de l'isomaltulose pour la préparation d'une composition pharmaceutique destinée à être utilisée dans un procédé pour améliorer la mémoire dans une phase de réponse post-glycémique d'un sujet malade ou perturbé mentalement qui en a besoin, dans laquelle la phase de réponse post-glycémique est de 180 minutes à 6 heures après la consommation de l'isomaltulose par le sujet qui en a besoin et est **caractérisée par** des niveaux de glucose dans le sang qui ne sont plus élevés après l'ingestion d'isomaltulose.
